# EUROPEAN PATENT SPECIFICATION

(11) **EP 2 694 130 B1**
(45) Date of publication and mention of the grant of the patent: **13.11.2019**
(21) Application number: 12767603.9
(22) Date of filing: 05.04.2012
(51) Int. Cl.: A61M 25/00, A61M 25/01, A61M 25/10

(54) **VARIABLE LENGTH CATHETER FOR DRUG DELIVERY**
KATHETER VON VARIABLER LÄNGE ZUR ABGABE VON ARZNEIMITTELN
CATHÉTER À LONGUEUR VARIABLE POUR ADMINISTRATION DE MÉDICAMENT

(30) Priority: 06.04.2011 US 201113080667; 02.06.2011 US 201113151378
(43) Date of publication of application: 12.02.2014
(73) Proprietor: Thermopeutix Inc., San Diego, California 92131 (US)
(72) Inventor: SOLAR, Ronald, Jay, San Diego CA 92131 (US); SHAKED, Yoav, 40695 Moshav Mishmeret (IL); LIEBER, Glen, Poway CA 92064 (US); DIPPEL, Eric, J., Davenport IA 52803 (US)
(74) Representative: White, Duncan Rohan
(86) International application number: PCT/IB2012/051713
(87) International publication number: WO 2012/137177

(56) References cited:
- WO-A1-2010/141500
- WO-A2-2006/081288
- US-A- 5 769 814
- US-A- 5 779 673
- US-A1- 2002 044 907
- US-A1- 2003 023 230
- US-A1- 2003 208 222
- US-A1- 2005 059 930
- US-A1- 2010 179 466

## Description

### FIELD AND BACKGROUND OF THE INVENTION

The present disclosure relates to systems and methods for delivery of therapeutic or diagnostic agents, wherein the systems provide for varying lengths of the treatment area. The systems and methods herein are designed for selective treating of an area without adversely affecting other parts of the body.

Methods and devices designed to provide drugs to a vessel, include, for example, the use of drug coated balloons, such as disclosed in US Patent Number 5,954,706 to Sahatjian, for example. Such devices include a catheter with an expandable portion, wherein at least a portion of the exterior surface of the expandable portion is defined by a coating of hydrogel polymer. Incorporated within the hydrogel polymer is a solution of a preselected drug to be delivered to the tissue or plaque. Disadvantages of such devices include the need to choose a particular drug and dosage in advance, as well as limitations on the length and diameter of the treatment area as defined by the predetermined length and diameter of the expandable portion, since these devices often work by direct contact of the device to the vessel.

Another device is disclosed in US Patent Number 6,287,320 to Slepian. A catheter includes first and second expansile members which are expanded to occlude a diseased region, and a therapeutic agent is introduced into the diseased region via the catheter. The catheter is allowed to remain in place for a therapeutically effective amount of time to allow the therapeutic agent to contact the diseased portion for such a period of time.

Another device is disclosed in US Patent Publication 2007/0078433 to Schwager et al. This device includes a balloon catheter having a predetermined inflow angle of medication. A first and second balloon are positioned on the catheter, with a treatment zone therebetween. Disadvantages of devices such as the ones disclosed in the above-referenced publications include limitations on the length of the treatment area as predetermined by the distance between the expansile members.

A device disclosed in US Patent Application Publication Number 2005/0059930 to Garrison et al. includes a catheter system with at least two expandable occluding elements which are used to create a localized site for administration of agents. The catheters are slidable with respect to one another to vary the space between the balloons as desired. However, the localized site is prone to overpressure since there is no disclosed way to remove excess fluid from the site.

In certain clinical applications, particularly in treating blood vessels, there may be small collateral vessels located within the desired drug treatment zone, and this may result in leakage of the agent from the zone. Outflow of blood distal to the occlusion site can provide a driving force to enhance the flow through these collateral vessels, and thus enhance the leakage of the agent from the desired treatment zone. There is thus a need for, and it would be highly advantageous to have, a system and method for localized drug delivery within a vessel, with adjustability of the length of the treatment area, with means for reducing pressure buildup in the treatment zone, and with ability to slow the distal outflow.

WO-A-2010/141500 discloses a catheter which may have optional pressure sensing elements which may allow for control of the pressure within the treatment zone and also prevent the pressure from exceeding a pressure of the inflatable/expandable members to thereby contain the treatment area between these inflatable/expandable members.

WO-A-2006/081288 discloses systems and methods for selective cooling or heating of a target site in the human body include a catheter having a supply elongated element and a delivery elongated element, with inlet and exit ports. Blood is withdrawn from the supply elongated element and cooled or heated in a control unit. The treated blood is sent to the targeted area via delivery elongated element.

### SUMMARY OF THE INVENTION

The invention provides a catheter system in accordance with claim 1. Any references to an "embodiment" or "embodiments" throughout the description which are not under the scope of the defined claims merely represent possible example(s) and are therefore not part of the present invention.

A core wire extends from the distal end to the proximal end of the inner elongated element. In accordance with further features of the present invention, the system may further include a treatment or diagnostic solution positioned within the outer elongated element lumen, between an outer wall of the inner elongated element and an inner wall of the outer elongated element and between the distal and proximal occlusion elements. Buildup of pressure by introduction of the solution can be prevented by blood flow through a pressure relief element at the distal end of the inner elongated element. The inner elongated element may further include a guidewire therethrough. A hub may be included at the outer elongated element proximal end, for introduction of a therapeutic or diagnostic solution into the outer elongated element lumen, and further for introducing inflation fluid to one or both of the occlusion elements. In some embodiments, the inner elongated element is introduced into the outer elongated element via the hub. The pressure relief element may be included either as a separate distal element or as an opening within the distal occlusion element, wherein the pressure relief element may be used for clearing blood out of the treatment zone, and additionally for introduction of a guidewire therethrough, for rapid exchange of catheters. In one embodiment the distal and proximal occlusion elements are inflatable balloons, and outer and inner elongated elements further comprise inflation lumens for inflating the inflatable balloons. In some embodiments, an inflow occlusion element is positioned proximal to a distal end of the proximal occlusion element. The inflow occlusion element may be a proximal part of the proximal occlusion element, or may be one or multiple separate occlusion elements placed proximal to the proximal end of the proximal occlusion element.

In accordance with further features of the present invention, the inner elongated element has an exposed portion, with an exposed portion proximal edge at a proximal occlusion element distal end, and an exposed portion distal edge at a distal occlusion element proximal end. The exposed portion has a length extending from the exposed portion proximal edge to the exposed portion distal edge, and this length may be varied by moving the inner elongated element with respect to the outer elongated element.

In accordance with another embodiment of the present invention, means are provided to occlude the blood vessel at a location proximal to the proximal occlusion element. The location, which is an inflow occlusion point, is proximal to a point in the vessel that provides a side-branch for blood flow, wherein the side-branch may communicate with collateral vessels that may be located within the treatment zone.

In accordance with an additional example, there is provided a method for treating a vessel. The method includes introducing an outer elongated element having a proximal occlusion element at a distal end thereof into the vessel, introducing an inner elongated element having a distal occlusion element and a pressure relief element at a distal end thereof, positioning the outer elongated element coaxially with respect to the inner elongated element, and positioning a distal end of the inner elongated element distal to the distal end of the outer elongated element. The distal occlusion element is positionable at varying distances from the proximal occlusion element. The method further includes deploying the distal and proximal occlusion elements and introducing a treatment or diagnostic solution through the outer elongated element into the vessel between the distal and proximal occlusion elements while simultaneously removing blood from the vessel via the pressure relief element. The method may further include occlusion of the blood vessel at a location proximal to the proximal occlusion element

In accordance with further features of the present disclosure, positioning of the outer elongated element coaxial to the inner elongated element may be done prior to introducing the outer and inner elongated elements into the vessel, or may be done during the introducing, wherein the inner elongated element may be introduced through the outer elongated element lumen, for example. The introducing into the vessel may be done by positioning the catheter over a guidewire positioned within the outer elongated element lumen, or by placing a guidewire through a pressure relief element. The distance between the distal and proximal occlusion elements may be adjusted prior to introducing the drug solution.

In accordance with an embodiment of the present invention, there is provided a system having an outer elongated element with an outer elongated element proximal end and an outer elongated element distal end, the outer elongated element having an outer elongated element lumen extending from the outer elongated element proximal end to the outer elongated element distal end and having an outlet port at the outer elongated element distal end, and a proximal occlusion element located at the outer elongated element distal end, the proximal occlusion element proximal to the outlet port, an inner elongated element having an inner elongated element proximal end and an inner elongated element distal end, the inner elongated element having an inner elongated element lumen extending from the inner elongated element proximal end to the inner elongated element distal end, a pressure relief element at the inner elongated element distal end, and a distal occlusion element located at the inner elongated element distal end. The inner elongated element is positioned within the outer elongated element lumen wherein the outer elongated element is coaxially arranged with respect to the inner elongated element, and wherein the inner elongated element distal end is distal to and movable with respect to the outer elongated element distal end. The system further may include a supply elongated element coaxial to the outer elongated element, the supply elongated element having an inlet port at a distal end thereof, wherein the inlet port is proximal to the outer elongated element distal end.

Unless otherwise defined, all technical and scientific terms used herein have the same meaning as commonly understood by one of ordinary skill in the art to which this invention belongs. Although materials similar or equivalent to those described herein can be used in the practice or testing of the embodiments of the present invention, suitable materials are described below. In case of conflict, the patent specification, including definitions, will control.

### BRIEF DESCRIPTION OF THE DRAWINGS

The invention is herein described with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1A is a schematic illustration of a system in accordance with examples of the disclosure, having an outer elongated element with a proximal occlusion element positioned thereon, an inner elongated element with a distal occlusion element positioned thereon, and at least one outlet port for delivery of a solution;
FIG. 1B, is a cross-sectional illustration of the system of FIG. 1A.
FIG. 1C is a schematic illustration of the system of FIG. 1A in accordance with another example of the present disclosure, wherein the system has multiple outlet ports;
FIG. 1D is a schematic illustration of the system of FIG. 1A in accordance with yet another example of the present disclosure, wherein the system has multiple outlet ports and an outlet port at a distal end of the proximal occlusion element;
FIG. 2 is an illustration of a system having a catheter and a supply lumen, in accordance with another example of the present disclosure;
FIGS. 3A-3F are schematic and cross-sectional illustrations of the system of FIGS. 1A-1D, further including a pressure relief element, in accordance with embodiments of the present invention;
FIG. 4 is a schematic illustration of a catheter with a fixed wire balloon, in accordance with embodiments of the present invention;
FIGS. 5A-5C are schematic illustrations of the system of FIGS. 1A-1D, showing a distal end thereof in accordance with examples of the present disclosure;
FIGS. 6A-6C are schematic illustrations of a system having a catheter in accordance with additional examples of the present disclosure, including at least one inflow occlusion element;
FIG. 7 is a schematic illustration of a system having a catheter in accordance with additional examples of the present disclosure, wherein the proximal occlusion element includes an inflow occlusion element;
FIGS.8A-8F are schematic illustrations of steps of a method of delivering a therapeutic or diagnostic agent to a treatment area of a vessel, in accordance with examples of the present disclosure,
FIGS. 9A-9D are schematic illustrations of steps of a method of delivering a therapeutic or diagnostic agent to a treatment area of a vessel with the catheter system of the present invention.
FIGS. 10A-10C are schematic illustrations of steps of a method of stopping outflow of a delivery substance using an inflow occlusion element, in accordance with examples of the present disclosure, and

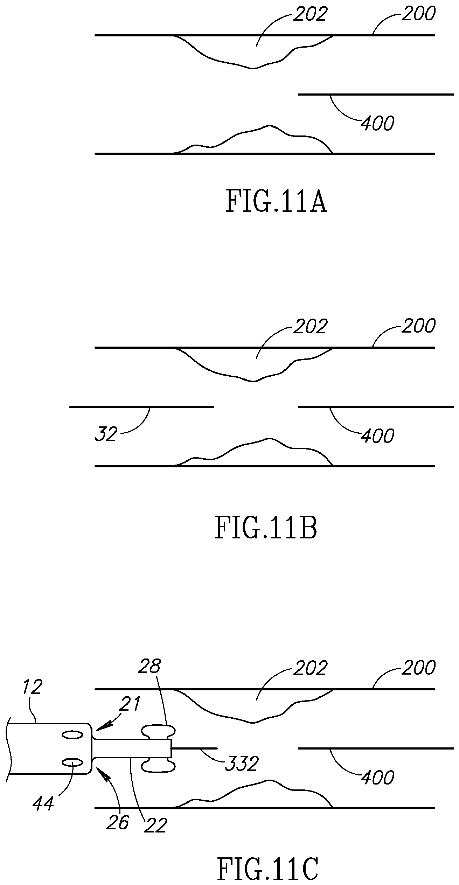

FIGS. 11A-11F are schematic illustrations of steps of a method of using a catheter in accordance with examples of the present disclosure for retrograde access of an artery such as a pedal artery. It will be appreciated that for simplicity and clarity of illustration, elements shown in the drawings have not necessarily been drawn accurately or to scale. For example, the dimensions of some of the elements may be exaggerated relative to other elements for clarity or several physical components may be included in one functional block or element. Further, where considered appropriate, reference numerals may be repeated among the drawings to indicate corresponding or analogous elements. Moreover, some of the blocks depicted in the drawings may be combined into a single function.

### DETAILED DESCRIPTION OF THE INVENTION

The present invention is of systems which can be used for delivery of a therapeutic or diagnostic agent into a vessel. For the purposes of the present invention, the term "delivery substance" is used to include any therapeutic or diagnostic agent which may be delivered into the vessel, including but not limited to medications, saline, contrast media, sealing agents, etc. The present invention provides a device wherein a user can selectively adjust the length of the vessel-access delivery portion of the catheter and can control the period of exposure of the delivery substance at the selected location in a contained manner.

The principles and operation of systems according to the present invention may be better understood with reference to the drawings and accompanying descriptions.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

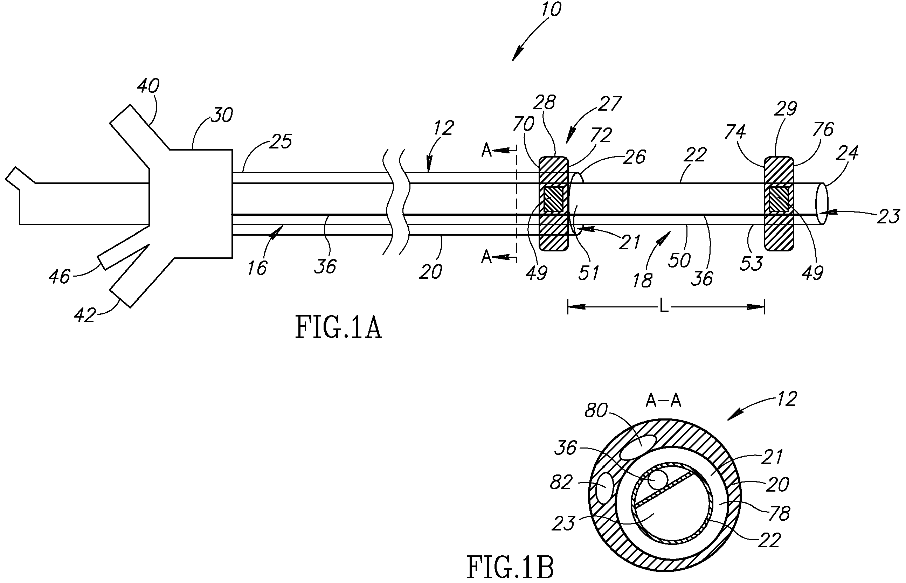

Reference is now made to FIGS. 1A and 1B, which are a schematic illustration and a cross-sectional illustration, respectively, of a system 10: System 10 includes a catheter 12 which includes an outer elongated element 20 having an outer elongated element lumen 21 therethrough and an inner elongated element 22 having an inner elongated element lumen 23 therethrough. Inner elongated element 22 is preferably an elongated tubular member, extending through an entire length of catheter 12, having an inner elongated element proximal end 16 and an inner elongated element distal end 18. In some embodiments, inner elongated element 22 has a guidewire exit port 24 at or near inner elongated element distal end 18. Guidewire exit port 24 is configured for placement of a guidewire therethrough, as will be explained further hereinbelow, but can also be used for perfusion, or exchange of different sized guidewires, for example. In some embodiments, inner elongated element lumen 23 and guidewire exit port 24 are not included, and the profile of inner elongated element 22 may be reduced. Outer elongated element 20 is preferably an elongated tubular member having an outer elongated element proximal end 25 and an outer elongated element distal end 27. Outer elongated element 20 is positioned externally and coaxially with respect to inner elongated element 22, as shown in cross-section A-A, in FIG. 1B, and extends from inner elongated element proximal end 16 to a location proximal to inner elongated element distal end 18.

As shown in FIG. 1A, outer elongated element 20 has an outlet port 26 located at outer elongated element distal end 27. In this embodiment, outlet port 26 is created by the coaxial arrangement of outer elongated element 20 and inner elongated element 22, wherein an inner diameter of outer elongated element 20 is sized at least 0.10 mm (i.e. 0.004") greater than an outer diameter of inner elongated element 22 and may be 3 mm (0.12") greater or more, depending on the size of catheter 12. The space created by this difference in diameter creates a delivery lumen 78 (depicted in FIG. 1B) which is sufficiently sized for providing a delivery substance to the vessel, as will be described in greater detail hereinbelow. A distal end of delivery lumen 78 is outlet port 26. Moreover, delivery lumen 78 may be sized for placement of a guidewire therethrough, as will be explained. In embodiments of the present invention, an outer diameter of inner elongated element 22 is in a range of 0,508 to 2,032 mm (0.02" to 0.08 inches), and more specifically may be in a range of 0,635 to 0,889 mm (0.025-0.035") for a smaller version of system 10 or in a range of 1,27 to 1,524 mm (0.05-0.06") for a larger version of system 10. An inner diameter of inner elongated element 22 is in a range of 0,254 to 1,27 mm (0.01" to 0.05") and more specifically may be in a range of 0,381 to 0,508 mm (0.015"-0.02") for a smaller version of system 10 and 0,889 to 1,143 mm (0.035"-0.045") for a larger version of system 10. An outer diameter of outer elongated element 20 is in a range of 0,127 to 0,381 mm (0.05"-0.15"), and more specifically may be in a range of 1,397 to 1,651 mm (0.055"-0.065") for a smaller version of system 10 and in a range of 2,921 to 3,302 mm (0.115"-0.130") for a larger version of system 10. An inner diameter of outer elongated element 20 is in a range of 0,635 to 2,54 mm (0.025" to 0.1"), and more specifically may be in a range of 0,762 to 1,27 mm (0.030"-0.050") for a smaller version of system 10 and in a range of 2,032 to 2,286 mm (0.080" to 0.090") for a larger version of system 10. It should be readily apparent that the shown example is not limited to the dimensions listed herein and that these dimensions should be taken as exemplary.

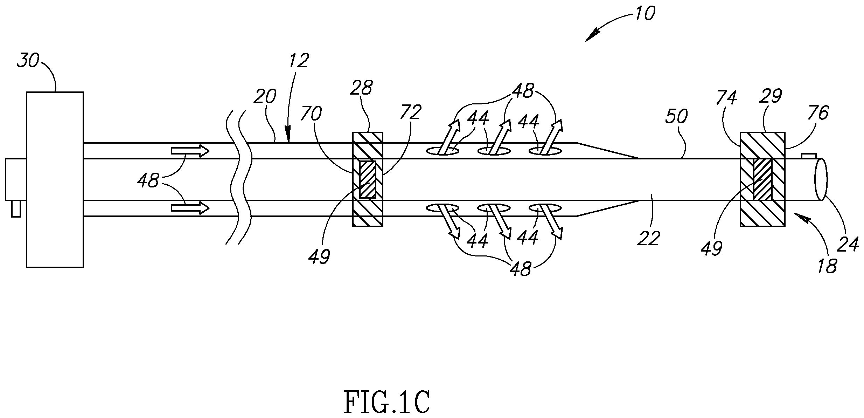

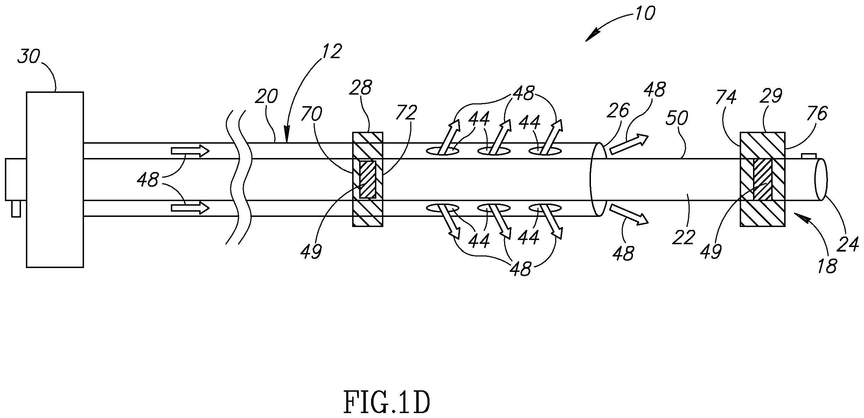

In another embodiment, as shown in FIG. 1C, outer elongated element 20 has multiple outlet ports 44, which are openings within a body of outer elongated element 20, at one or more locations along its length, for providing a delivery substance to the blood vessel. Outward movement of drug solution, contrast, diagnostic solution, or other substance from proximal end 16 to and through multiple outlet ports 44 is indicated by arrows 48. In yet another embodiment, as shown in FIG. 1D, both outlet port 26 and multiple outlet ports 44 are used, and the substance may exit through both or through either of outlet port 26 and multiple outlet ports 44.

Returning now to FIG. 1A, a proximal occlusion element 28 is positioned on outer elongated element 20 at or near outer elongated element distal end 27, such that proximal occlusion element 28 is proximal to outlet port 26. In the embodiments shown in FIGS. 1C and 1D, proximal occlusion element 28 is positioned proximal to multiple outlet ports 44. Proximal occlusion element 28 has a proximal occlusion element proximal end 70 and a proximal occlusion element distal end 72. A distal occlusion element 29 is positioned on inner elongated element 22, at or near inner elongated element distal end 18, proximal to guidewire exit port 24, and distal to outer elongated element distal end 27. Distal occlusion element 29 has a distal occlusion element proximal end 74 and a distal occlusion element distal end 76. Inner elongated element 22 is movable with respect to outer elongated element 20. Thus, an exposed portion 50 of catheter 12 may be defined as having an exposed portion proximal edge 51 at proximal occlusion element distal end 72 and an exposed portion distal edge 53 at distal occlusion element proximal end 74. Exposed portion 50 has a length L extending from exposed portion proximal edge 51 to exposed portion distal edge 53, and length L may be varied by moving inner elongated element 22 with respect to outer elongated element 20.

Inner elongated element lumen 23 may be configured to hold a guidewire therein, and outer elongated element lumen 21 is configured to hold inner elongated element 22 therein and to further hold a delivery substance in between an outer wall of inner elongated element 22 and an inner wall of outer elongated element 20 within delivery lumen 78. In some embodiments, outer elongated element lumen 21 is further configured to hold a guidewire therein. The delivery substance may be introduced into the vessel through outlet port 26 and/or multiple outlet ports 44, but is prevented from flowing outside of a treatment zone by inflation of proximal occlusion element 28 and inflation of distal occlusion element 29. Thus, a length of the treatment zone in the vessel is determined by length L of exposed portion 50.

A hub 30 is positioned at a proximal end of catheter 12 and is attached to outer elongated element 20 at outer elongated element proximal end 25. Hub 30 includes an infusion port 40 for introducing a delivery substance such as a drug solution into delivery lumen 78 (i.e. outer elongated element lumen 21) and a proximal occlusion element inflation port 42 for delivery of inflation fluid to proximal occlusion element 28. Hub 30 may further include a pressure monitoring valve 46. Hub 30 may also include a distal occlusion element inflation port (not shown).

Referring now to FIG. 1B, the configuration of outer elongated element 20 and inner elongated element 22 is shown in cross-section. Outer elongated element lumen 21 is configured to receive therein both inner elongated element 22 and a delivery substance introduced via infusion port 40. Outer elongated element 20 may further include an inflation lumen 80 for introducing inflation fluid into proximal occlusion element 28, and in some embodiments further includes a pressure lumen 82. Pressure lumen 82 has a proximal pressure transducer attached thereto which is capable of measuring the pressure of a column of fluid located within pressure lumen 82. Outer elongated element lumen 21 may also be configured to receive a guidewire therethrough, in between the body of outer elongated element 20 and inner elongated element 22. Inner elongated element 22 may have an inner elongated element lumen 23 for receiving a guidewire therethrough, and further includes a distal inflation lumen for introducing inflation fluid into distal occlusion element 29. A core wire 36 is positioned within or attached to inner elongated element 22. In some embodiments, core wire 36 is positioned between layers of a polymer shaft of inner elongated element 22.

Radiopaque markers 49 may be included on distal occlusion element 29, proximal occlusion element 28 and other locations along catheter 12 for visualization of the position of catheter 12 within the vessel and relative positions of distal and proximal occlusion elements 29 and 28. In a preferred embodiment, radiopaque markers 49 are located at the distal end of proximal occlusion element 28 and the proximal end of distal occlusion element 29.

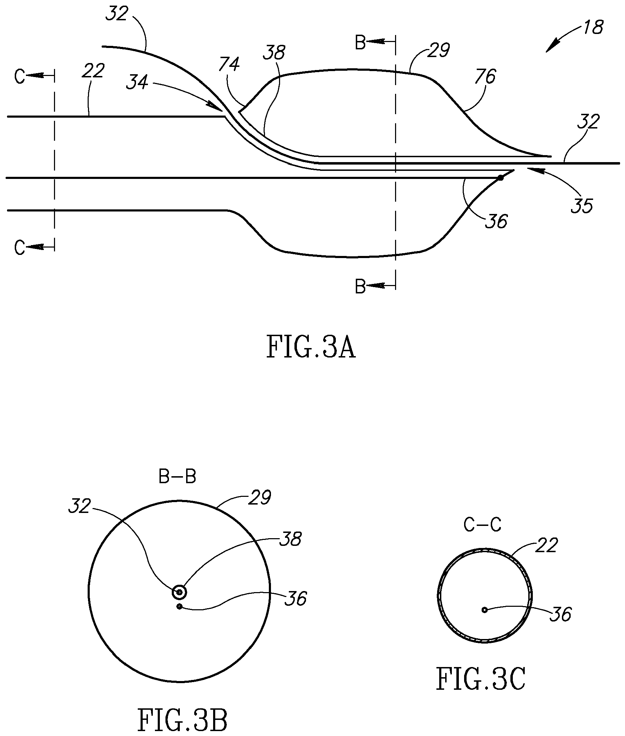

In some embodiments, outer elongated element 20 is advanced into a vessel first with a guidewire positioned through outer elongated element lumen 21, followed by advancement of inner elongated element 22 which may be advanced through outer elongated element lumen 21 of outer elongated element 20, resulting in the guidewire and inner elongated element 22 positioned side by side within outer elongated element lumen 21. In other embodiments, outer elongated element 20 and inner elongated element 22 are advanced together into the vessel. The introduction of system 10 with outer elongated element 20 and inner elongated element 22 may be done either as an over the wire system, wherein a guidewire is introduced into the vessel and then positioned within inner elongated element lumen 23, whereupon system 10 is advanced over the guidewire, or may be done using a pressure-relief element on inner elongated element 22, as will be described below with reference to FIGS. 3A-3F.

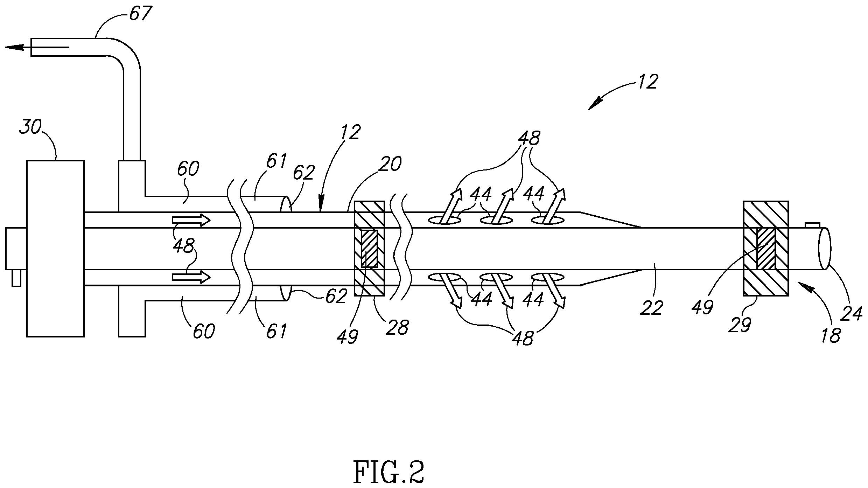

Reference is now made to FIG. 2, which is an illustration of a system 10 having a catheter 12. Catheter 12 is similar in construction to catheter 12 shown in FIGS. 1A-1D, with an additional feature of a supply elongated element 60 positioned external and coaxial to inner and outer elongated elements 22 and 20. Supply elongated element 60 is configured for removing blood from the blood vessel during treatment.

In one embodiment, supply elongated element 60 has inlet ports at one or more locations along its length for receiving blood from the blood vessel. In a preferred embodiment, as shown in FIG. 2, supply elongated element 60 has an inlet port 62 located at a distal end 61 thereof. In this embodiment, inlet port 62 is created by the coaxial arrangement of supply elongated element 60 and outer elongated element 20, wherein an inner diameter of supply elongated element 60 is sized at least 0.1 mm greater than an outer diameter of outer elongated element 20. The space created by this difference in diameter creates a port which is sufficiently sized for receiving blood from the vessel, as will be described in greater detail hereinbelow.

Hub 30 may connect supply elongated element 60 and inner elongated element 22 to a control unit. The control unit may thermally alter (i.e. heat or cool) normothermic blood received from supply elongated element 60, and send the thermally altered blood into the blood vessel through inner elongated element 22. Blood received from supply elongated element 60 may be treated or altered in other ways as well, or may be used to perfuse the vessel distal to distal occlusion element 29.

In one embodiment, supply elongated element 60 is a standard vascular sheath and may have a side arm 67 from which blood is removed from the vessel and in some embodiments sent to a control unit. In another embodiment, supply elongated element 60 is an extended sheath, and may extend distally to 100 cm or more depending on the application.

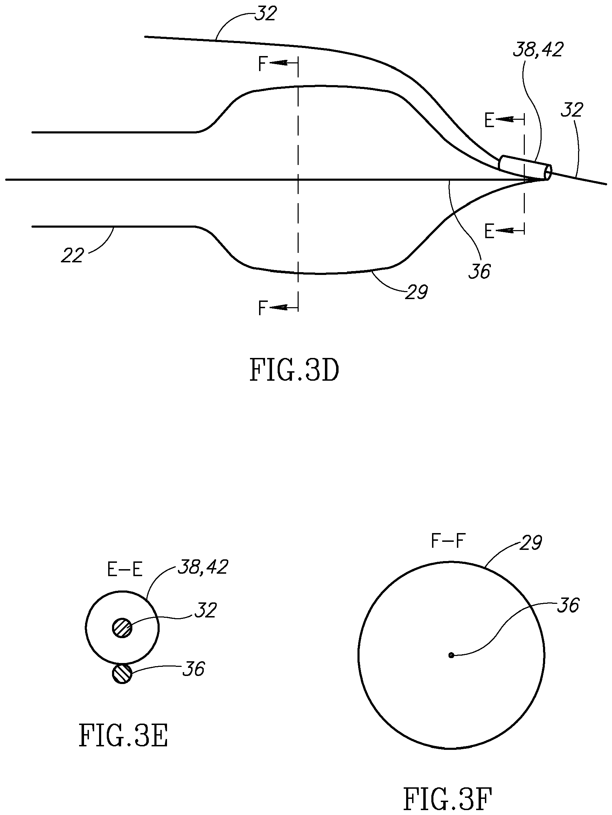

Reference is now made to FIGS. 3A-3F, which are schematic illustrations (FIGS. 3A and 3D) and cross-sectional illustrations (FIG. 3B, 3C, 3E and 3F), respectively, of inner elongated element 22 with a pressure relief element 38 in accordance with embodiments of the present invention. Pressure may build up in the vessel due to introduction of a delivery substance. As such, it would be beneficial to have means for reducing this pressure. Pressure relief element 38 provides controlled removal of blood from the treatment area to a location outside of the treatment area, as will be described hereinbelow. Pressure relief element 38 comprises a passageway for blood, and in some embodiments may also be used for placement therethrough of a movable guidewire 32. Pressure relief element 38 is sized with a diameter slightly larger than a diameter of movable guidewire 32. For example, an inner diameter of pressure relief element 38 may be approximately 0,0508 mm (0.002") greater than a diameter of movable guidewire 32. This difference in diameter provides a clearance space for controlled removal of blood from the treatment zone, which can be useful in preventing pressure buildup in the treatment zone when the delivery substance is introduced. In some embodiments, the clearance space is sized such that only blood can move through, and not the delivery substance, due to blood having a lower viscosity than the delivery substance. For example, contrast solution, which has a higher viscosity than blood, should not be able to move through the clearance space.

Core wire 36 is positioned within inner elongated element 22 and may be attached to distal occlusion element 29 at a distal end thereof and to proximal end 16 of inner elongated element 22. Core wire 36 may further be attached at additional points along the length of inner elongated element 22. In some embodiments, core wire 36 is sandwiched between polymeric layers of a shaft of inner elongated element 22.

In one embodiment, as shown in FIG. 3A, pressure relief element 38 comprises a passageway through distal occlusion element 29. In this embodiment, pressure relief element 38 replaces inner elongated element lumen 23. Instead of extending from a distal end to a proximal end of catheter 12, as inner elongated element lumen 23 would, pressure relief element 38 extends from a proximal opening 34 at distal occlusion element proximal end 74, to a distal opening 35 at distal end 18 of catheter 12. Movable guidewire 32 may be introduced into catheter 12 at inner elongated element distal end 18 through distal opening 35 and may exit at proximal opening 34 located at or near distal occlusion element proximal end 74. Reference is made to FIG. 3B, which is a cross-sectional illustration of catheter 12 of FIG. 3A, showing a cross-section at B-B. Distal occlusion element 29 is shown, with pressure relief element 38 positioned through distal occlusion element 29 and with movable guidewire 32 positioned within pressure relief element 38. Core wire 36 is depicted as well, positioned through distal occlusion element 29. Reference is made to FIG. 3C, which is a cross-sectional illustration of catheter 12 of FIG. 3A, showing a cross-section at C-C. At this more proximal portion of catheter 12, inner elongated element 22 has core wire 36 but does not include pressure relief element 38 or movable guidewire 32. Movable guidewire 32 is outside of inner elongated element 22 at this point, as shown in FIG. 3A.

In another embodiment, as shown in FIGS. 3D-3F, pressure relief element 38 comprises a separate distal element 42 positioned on inner elongated element 22 distal to distal occlusion element 29. In some embodiments, distal element 42 may have a length of 4-20 cm. Pressure relief element 38 allows for rapid exchange of catheters and for removal of blood from the treatment zone. Inner elongated element 22 further includes a core wire 36 positioned for providing stiffness through catheter 12. This enhances pushability of catheter 12. Core wire 36 is positioned within inner elongated element 22 and may be attached to distal occlusion element 29 at a distal end thereof and to proximal end 16 of inner elongated element 22. Core wire 36 may further be attached at additional points along the length of inner elongated element 22. In some embodiments, core wire 36 is sandwiched between polymeric layers of a shaft of inner elongated element 22. Reference is made to FIG. 3E, which is a cross-sectional illustration of catheter 12 of FIG. 3D, showing a cross-section at E-E. Pressure relief element 38 is shown, with movable guidewire 32 positioned therethrough. Core wire 36 is depicted as well, positioned outside of pressure relief element 38. Reference is made to FIG. 3F, which is a cross-sectional illustration of catheter 12 of FIG. 3D, showing a cross-section at F-F. At this more proximal portion of catheter 12, distal occlusion element 29 is shown with core wire 36 positioned therethrough. Movable guidewire 32 is outside of inner elongated element 22, as shown in FIG. 3D.

Pressure relief element 38 may provide added advantages because it provides an additional lumen within system 10. For the embodiments shown in FIGS. 3A-3F wherein a pressure relief element 38 is used with movable guidewire 32, outer elongated element lumen 21 may house movable guidewire 32 when inner elongated element 22 and outer elongated element 20 are positioned coaxially to one another. In these embodiments, inner elongated element 22 and outer elongated element 20 may be initially positioned coaxial to one another, and movable guidewire 32 is introduced through pressure relief element 38 of inner elongated element 22. Catheter 12, having both inner and outer elongated elements 22 and 20, is advanced over movable guidewire 32. Once movable guidewire 32 is positioned within pressure relief element 38, movable guidewire 32 is further positioned in between an outer surface of inner elongated element 22 and an inner surface of outer elongated element 20 - that is, within outer elongated element lumen 21. This positioning allows for an over the wire type of advancement, but with a reduced profile, since an additional over the wire lumen is not required. In this case, inner elongated element lumen 23 may be eliminated thus reducing the profile of catheter 12.

In some embodiments, inner elongated element lumen 23 may be maintained and used for other items even when not in use for movable guidewire 32. For example, a mandrel may be introduced through inner elongated element lumen 23 for enhancing pushability and for advancing inner elongated element 22. In some embodiments, inner elongated element lumen 23 may be used for exchanging guidewires, or for putting a second guidewire in the vessel. Alternatively, inner elongated element lumen 23 may be used for perfusion. For example, in a case of prolonged occlusion while treating the vessel, blood may be introduced through inner elongated element lumen 23 to an area distal to distal occlusion element 29, thus making it possible to keep treating the vessel for as long as necessary. This may be particularly useful in the coronary arteries, for example, which cannot be occluded for a prolonged period of time. In some embodiments, blood may be cooled or otherwise treated and then introduced through inner elongated element lumen 23. In some embodiments, a supply elongated element is included as well, as described with reference to FIG. 2, for removing blood from the vessel which may then be reintroduced through inner elongated element lumen 23. In some embodiments, inner elongated element 22 may be removed from outer elongated element 20 during a procedure.

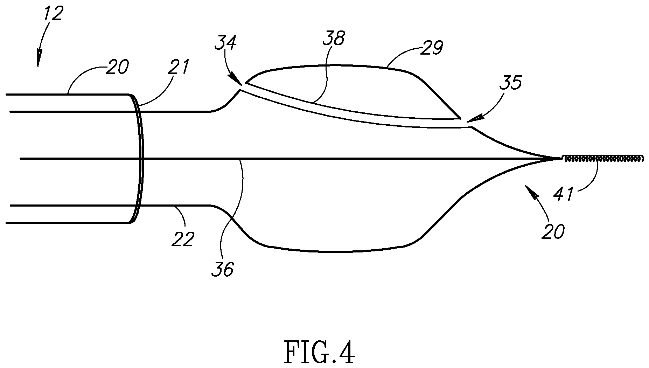

Reference is now made to FIG. 4, which is an illustration of a catheter 12 in accordance with yet another embodiment. In this embodiment, pressure relief element 38 has a smaller diameter and may not be used for placement of a movable guidewire therethrough. For example, pressure relief element 38 may have a diameter of 0,0254 to 0,0508 mm (0.001" to 0.002") - enough for blood to flow through, but not enough for a delivery substance due to its higher viscosity, and not enough for a movable guidewire. In this embodiment, a fixed wire 41 may be positioned at distal end 18 of inner elongated element 22. Thus, for example, distal occlusion element 29 may be a fixed wire balloon. In some embodiments, an additional movable wire may be introduced through outer elongated element lumen 21. In yet another embodiment, a pressure relief element 38 is sized for a movable guidewire and in addition a fixed wire 41 is used as well.

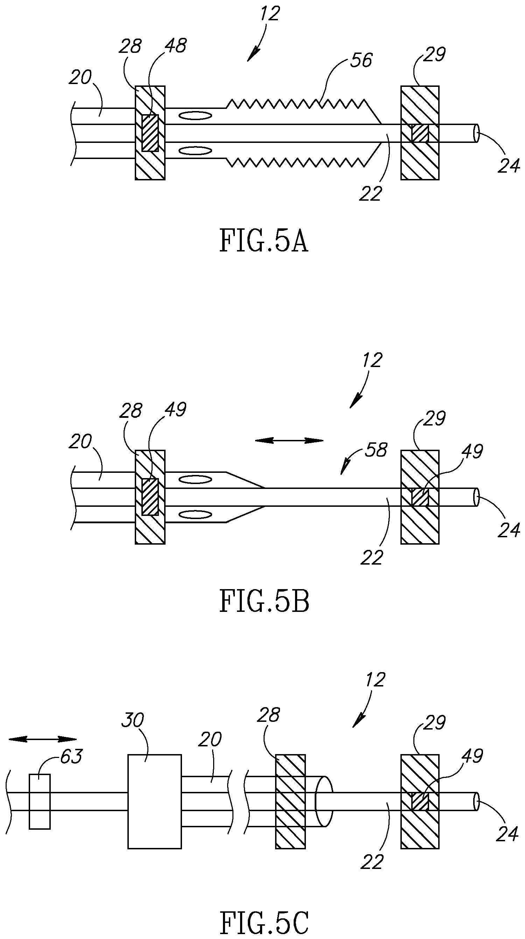

Reference is now made to FIGS. 5A-5C, which are illustrations of a distal portion of catheter 12, in accordance with examples of the present disclosure, wherein distal occlusion element 29 is positionable at varying distances from proximal occlusion element 28. As described above, inner elongated element 22 is movable with respect to outer elongated element 20. Movement can be a twisting motion, for example, wherein inner elongated element 22 and outer elongated element 20 are attached with a bellows 56, as shown in FIG. 5A. Alternatively, movement can be a sliding motion, wherein inner elongated element 22 and outer elongated element 20 are attached via telescoping means 58, as shown in FIG. 5B. In a preferred embodiment, movement is achieved by coaxial arrangement of outer elongated element 20 and inner elongated element 22, as shown in FIG. 5C. In this arrangement, it may be necessary to include an adjustable anchor 63 for anchoring the proximal portion of inner elongated element 22 to the body or surgical drape of the patient. Alternatively, a length of outer elongated element 20 may protrude proximal to the proximal end of catheter 12. In this case, it may be necessary to include an adjustable anchor for anchoring the proximal portion of outer elongated element 20 to the body or surgical drape of the patient. Any suitable adjustable anchor means may be used, including, for example, a luer lock, a gland, a squeeze-lock mechanism, etc. Any other means for changing a distance between distal occlusion element 29 and proximal occlusion element 28 or between distal end 18 of inner elongated element 22 and distal end 27 of outer elongated element 20 is included within the scope of the disclosure.

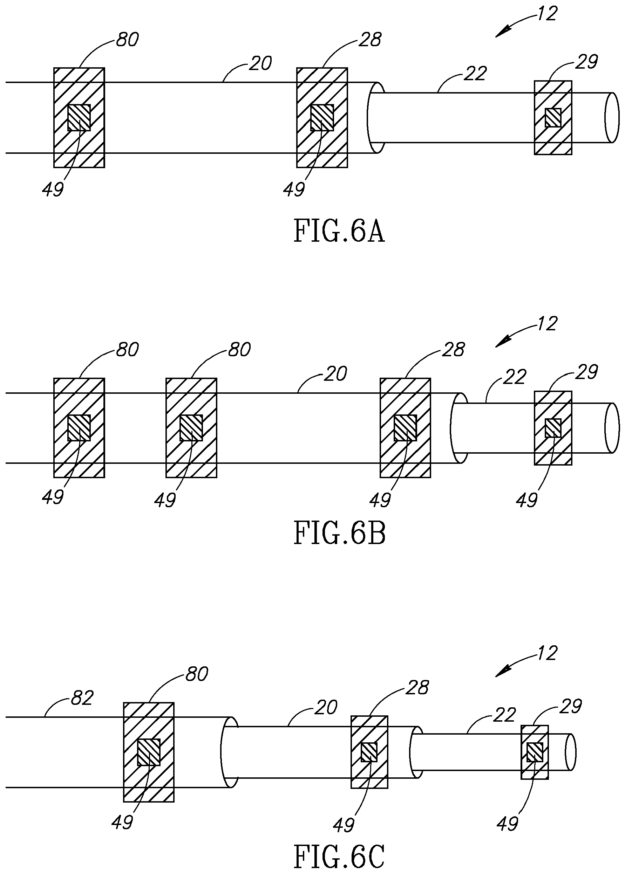

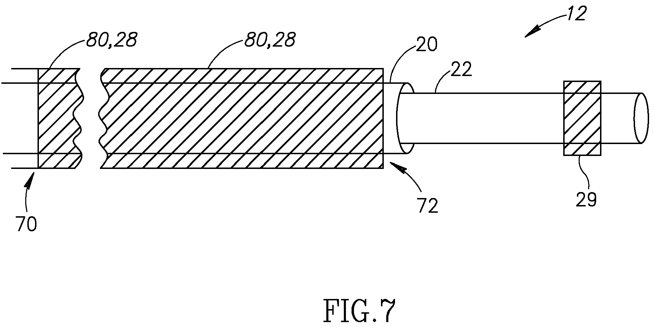

Reference is now made to FIGS. 6A-6C, and to FIG. 7 which are schematic illustrations of catheter 12 in accordance with additional examples of the present disclosure. In the embodiments shown herein, catheter 12 further includes one or multiple inflow occlusion elements 80 positioned proximal to proximal occlusion element 28. The vessel being treated may have a side-branch which runs alongside the treatment zone. Outflow of blood through collateral blood vessels connecting the side-branch with the main vessel may cause loss of delivery substance as well. It has been shown that by temporarily stopping inflow of blood at a point which is proximal to the side branch, this point being defined as an "inflow occlusion point", it is possible to eliminate or at least slow down outflow of blood from the treatment zone. By placing an inflow occlusion element 80 on a proximal portion of catheter 12, blood flow into collateral vessels, and consequently leakage of the delivery substance from the treatment zone, may be reduced.

In one embodiment, as shown in FIG. 6A, inflow occlusion element 80 is positioned proximal to proximal occlusion element 28 in a fixed position. In this embodiment, several versions of catheter 12 may be constructed, each having a predetermined distance between inflow occlusion element 80 and proximal occlusion element 28. A user can choose the appropriately designed catheter 12 for a particular application.

In another embodiment, as shown in FIG. 6B, multiple inflow occlusion elements 80 may be positioned at locations proximal to proximal occlusion element 28. A user may then choose to inflate any one, or all, of multiple inflow occlusion elements 80 to stop or reduce blood outflow at a particular location.

In another embodiment, as shown in FIG. 6C, inflow occlusion element 80 is positioned on an inflow occlusion shaft 82. Inflow occlusion shaft 82 is proximal to and coaxial to outer elongated element 20 and is movable with respect to outer elongated element 20. Thus, an optimal position of inflow occlusion element 80 may be found and may be adjusted prior to inflation of inflow occlusion element 80. Markers 49 may be included on inflow occlusion element 80.

In one embodiment, as shown in FIG. 7, inflow occlusion element 80 is an elongated version of proximal occlusion element 28. Thus, proximal occlusion element 28 may be elongated such that proximal occlusion element proximal end 70 is a distance from proximal occlusion element distal end 72. The distance between proximal occlusion element proximal end 70 and proximal occlusion element distal end 72 may be in a range of 5 cm - 40 cm, for example. It should be readily apparent that other ranges are possible as well, and that any distance which provides proximal occlusion element proximal end 70 at an inflow occlusion point is within the scope of the present disclosure. Thus, upon inflation of proximal occlusion element 28, proximal occlusion element distal end 72 defines the treatment zone, as described above, while at the same time, proximal occlusion element proximal end 70 provides inflow occlusion.

Proximal and distal occlusion elements 29 and 28 are comprised of an atraumatic surface so as not to damage the inner walls of a blood vessel. In a preferred embodiment, proximal and distal occlusion elements 29 and 28 have a hydrophilic surface, which by attracting water forms a natural atraumatic layer. Furthermore, a hydrophilic surface can provide means for occlusion which is configured to open when in contact with water components from the blood. Proximal and distal occlusion elements 29 and 28 may further include a coating for providing long-term (measured in hours, days or even months) implantation of catheter 12 in the body. Alternatively or in addition, proximal and distal occlusion elements 29 and 28 may further include a drug coating. In one embodiment, proximal and distal occlusion elements 29 and 28 are balloons, such as are commonly used with catheter systems, and are expandable by introduction of a fluid therein, wherein the fluid can be a liquid or a gas. In this embodiment, separate inflation lumens are included within catheter 12, either alongside or coaxial with delivery elongated element 22, and are in fluid communication with proximal and distal occlusion elements 29 and 28. Fluid is introduced via an inflation port (not shown) positioned at hub 30. These types of balloons and inflation lumens are commonly known in the art. The balloon may be elastomeric, compliant, semi-compliant or non-compliant, as long as it serves to occlude the vessel without causing damage to the internal walls. In one embodiment, the balloon is pre-formed and relatively thin, so as to reduce the pressure necessary to inflate the balloon, while keeping the outer diameter to a minimum. For example, balloon thickness may range from 0,00254 mm to 0,0508 mm (0.0001 inches to 0.002 inches) a range which is smaller than thicknesses of standard occlusion balloons.

In another embodiment, proximal and distal occlusion elements 29 and 28 are self-expanding elements confined within retractable sheaths, such that upon retraction of the sheath, the self-expanding element expands to a diameter sufficient to occlude the vessel. In this embodiment, the sheath is connected to a retractor positioned at proximal end 16 of catheter 12. The self-expanding element may be comprised of an elastic or spring-like material, or a shape-memory alloy. Such materials are known in the art. In another embodiment, proximal and distal occlusion elements 29 and 28 are comprised of a mechanically actuated mechanism, whereby they are expanded by mechanical means. In yet another embodiment, proximal and distal occlusion elements 29 and 28 are comprised of a temperature sensitive material which can be expanded or retracted by exposure to specific temperatures. Specifically, perfusion of cooled or heated blood through catheter 12 would cause expansion of proximal and distal occlusion elements 29 and 28 , and perfusion of normothermic blood through catheter 12 (such as, for example, during renormalization of temperature) would cause retraction of proximal and distal occlusion elements 29 and 28 . This may be accomplished, for example, by using a shape-memory material, either as proximal and distal occlusion elements 29 and 28 themselves, or as an actuator positioned alongside proximal and distal occlusion elements 29 and 28. Similarly, this could be accomplished by using a bi-metallic strip. In one embodiment, proximal and distal occlusion elements 29 and 28 are an integral part of the catheter, wherein a portion of catheter 12 having a slightly wider diameter is configured to be wedged into the vessel, and thus acts as occlusion element, providing both occlusion and anchoring functionality.

Proximal and distal occlusion elements 29 and 28 further include radiopaque markers 49 for viewing of a location of catheter 12 generally and proximal and distal occlusion elements 29 and 28 and 29 specifically within the vessel. In one embodiment, proximal and distal occlusion elements 29 and 28 are themselves comprised of radiopaque material. In alternative embodiments, one or more radiopaque markers 49 are positioned on or adjacent to proximal and distal occlusion elements 29 and 28. Additional radiopaque markers 49 may also be positioned in other places along catheter 12 such as, for example, at distal end 18. In one embodiment, a radiopaque marker 49 is positioned at the distal tip of catheter 12. Radiopaque marker 49 can be a ring surrounding the distal tip, or, in order to minimize stiffness at the tip, a radiopaque marker may be comprised of a small sliver of radiopaque material embedded within a portion of the distal tip. In one embodiment, radiopaque marker 49 is filled with an adhesive and positioned so as to seal an inflation lumen for inflation of proximal and distal occlusion elements 29 and 28 and 29.

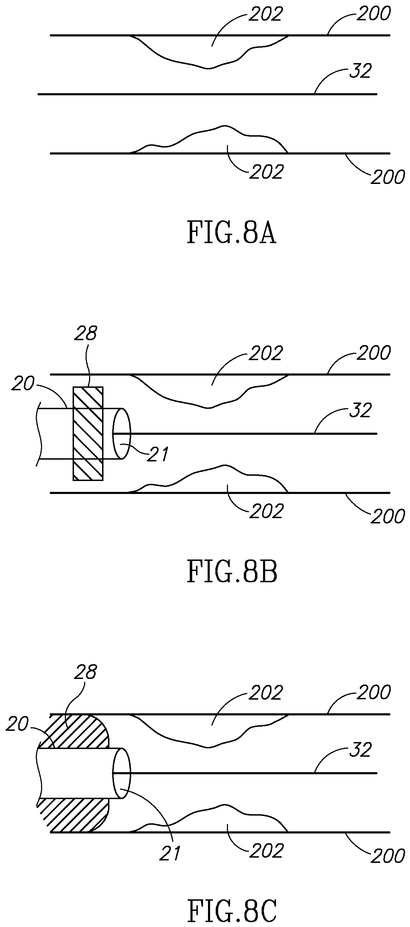

Reference is now made to FIGS. 8A-8F, which are schematic illustrations showing a method of using catheter 12.

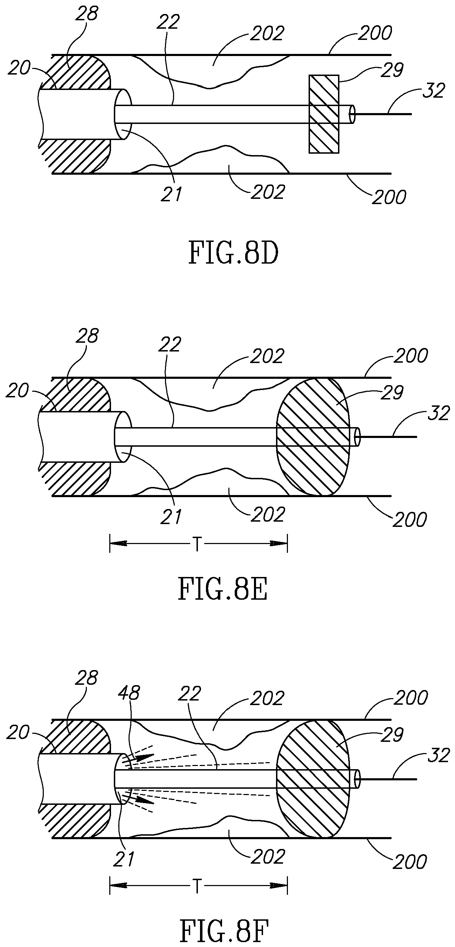

A vessel 200 is shown with a lesion 202. As shown in FIG. 8A, movable guidewire 32 is introduced into vessel 200 adjacent lesion 202. As shown in FIG. 8B, outer elongated element 20 is introduced over movable guidewire 32, and is positioned proximal to lesion 202. Next, as shown in FIG. 8C, proximal occlusion element 28 is inflated. In an alternative example, proximal occlusion element 28 is inflated later on in the procedure, after inner elongated element 22 is in place. Next, as shown in FIG. 8D, inner elongated element 22 is introduced through outer elongated element lumen 21 and is positioned distal to lesion 202. Next, as shown in FIG. 8E, distal occlusion element 29 is inflated, thus defining a treatment area T between inflated proximal occlusion element 28 and inflated distal occlusion element 29. Next, as shown in FIG. 8F, a delivery substance, such as a drug solution, is introduced into treatment area T via outer elongated element lumen 21 of outer elongated element 20. Introduction of delivery substance is depicted by arrows 48. It should be readily apparent that treatment area T may be adjusted by introducing inner elongated element 22 at varying distances from a distal end of outer elongated element 20. Alternatively or in addition to the embodiments shown herein, outer elongated element 20 may have one or multiple outlet ports 26, 44 as described with reference to FIGS. 1A, 1C and 1D. In some examples, the delivery substance may also be removed from vessel 200 through outlet port 26 and through lumen 21. In some examples, the method may include a repeatable cycle of introducing the delivery substance, removing the delivery substance, deflating distal and proximal occlusion elements, reestablishing blood flow, reinflating distal and proximal occlusion elements, and reintroducing the same or a different delivery substance. This cyclic introduction and removal of the delivery substance is possible since the delivery substance can remain inside outer elongated element 20, and distal and proximal occlusion elements 29 and 28 may be inflated and deflated. This method can provide a benefit of prolonged drug exposure without prolonged stoppage of blood flow. In some examples, inner elongated element 22 may be removed from vessel 200 during the procedure, and a different catheter or device may be introduced through outer elongated element 20 for additional procedures.

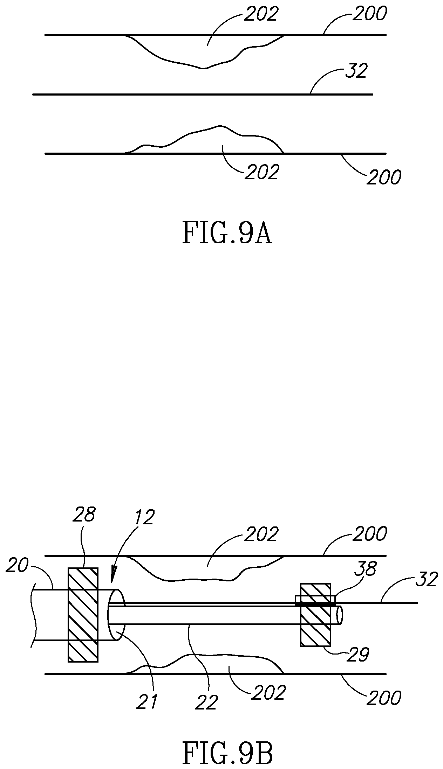

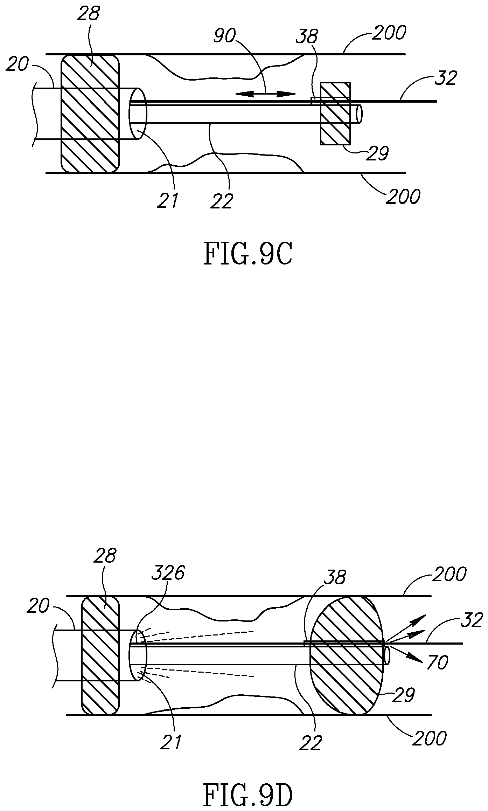

Reference is now made to FIGS. 9A-9D, which are schematic illustrations showing a method of using catheter 12 with the catheter system of the present invention. A vessel 200 is shown with a lesion 202. As shown in FIG. 9A, movable guidewire 32 is introduced into vessel 200 adjacent lesion 202. Next, as shown in FIG. 9B, catheter 12 having inner elongated element 22 positioned within outer elongated element 20, is introduced over movable guidewire 32 by placing movable guidewire 32 through pressure relief element 38 on inner elongated element 22. Movable guidewire 32 is further positioned proximally through outer elongated element lumen 21 of outer elongated element 20. Next, as shown in FIG. 9C, proximal occlusion element 28 is inflated, and inner elongated element 22 is adjusted, as shown by arrows 90, such that distal occlusion element 29 is positioned distal to lesion 202. Once inner elongated element 22 is in position, distal occlusion element 29 may be inflated, as shown in FIG. 9D. Blood may be allowed to leak out through pressure relief element 38, as depicted by arrows 70, and a delivery substance, such as a drug solution, is introduced from outside of catheter 12 into outer elongated element lumen 21 of outer elongated element 20 and into vessel 200 through outlet port 26 and/or through multiple outlet ports 44 (not shown). Alternatively, the sequence of balloon inflation may be varied. For example, distal occlusion element 29 may be inflated first, followed by proximal occlusion element 28. It should be readily apparent that a feature of the present invention is the flexibility in inflating and/or deflating the occlusion elements as necessary. In some examples, the delivery substance may also be removed from vessel 200 through outlet port 26. In some examples, the method may include a repeatable cycle of introducing the delivery substance, removing the delivery substance, deflating distal and proximal occlusion elements, reestablishing blood flow, reinflating distal and proximal occlusion elements, and reintroducing the same or a different delivery substance. This cyclic introduction and removal of the delivery substance is possible since the delivery substance can remain inside outer elongated element 20, and distal and proximal occlusion elements may be inflated and deflated. This method can provide a benefit of prolonged drug exposure without prolonged stoppage of blood flow. In some examples, inner elongated element 22 may be removed from vessel 200 during the procedure, and a different catheter or device may be introduced through outer elongated element 20 for additional procedures.

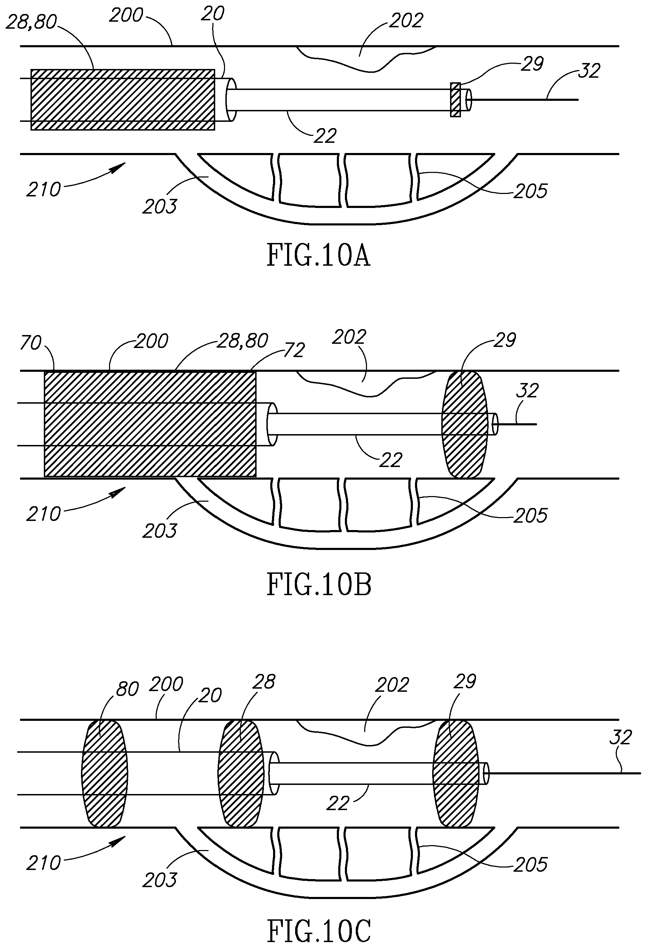

Reference is now made to FIGS. 10A-10C, which are schematic illustrations of methods of using catheter 12. As shown in FIG. 10A, catheter 12 is inserted into a vessel 200 over movable guidewire 32, and is positioned with proximal occlusion element 28 proximal to lesion 202 and with distal occlusion element 29 distal to lesion 202. In this example, a side branch vessel 203 runs alongside vessel 200, with collateral blood vessels 205 connecting vessel 200 to side branch vessel 203. The vessel 200 may be, for example, the superficial femoral artery. Since the side branch vessel 203 is adjacent to a treatment area T, blood flow through the side branch vessel 203 may cause outflow from the vessel 200 through the collateral blood vessels 205. Thus, in examples of the methods shown herein, blood outflow to side branch vessel 203 is occluded in order to eliminate or slow down blood outflow from the collateral blood vessels 205. Next, as shown in FIG. 10B, proximal occlusion element 28 is inflated and distal occlusion element 29 is inflated, thus defining a treatment area T between inflated proximal occlusion element 28 and inflated distal occlusion element 29. In this example proximal occlusion element 28 also includes inflow occlusion element 80 , which is inflated to prevent inflow of blood into an inflow occlusion point 210 located proximal to a point in the vessel that provides a side-branch to the vessel leading to the treatment zone. In another example, as shown in FIG. 10C, inflow occlusion element 80 is a separate occlusion element and is inflated at or just proximal to inflow occlusion point 210. It should be readily apparent that further steps for providing a delivery substance to a treatment area are also performed, as described above.

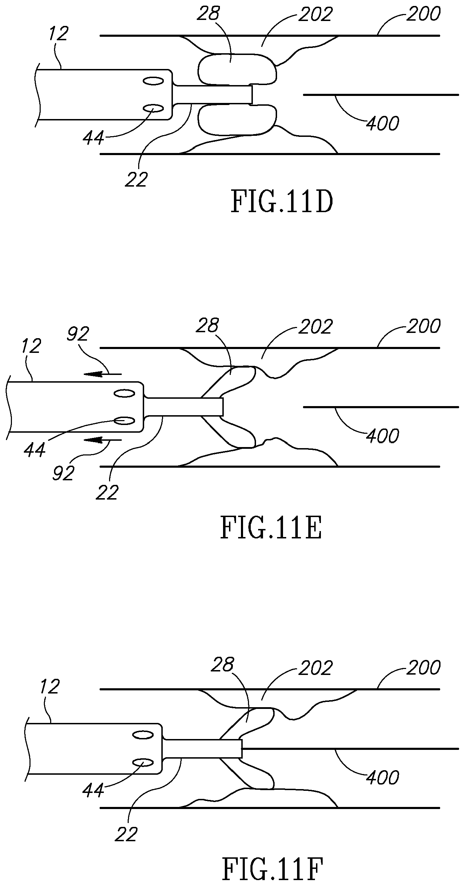

Reference is now made to FIGS. 11A-11F, which are schematic illustrations of a method of using catheter 12 for retrograde access to a lesion, from, for example, an artery such as a pedal artery. In this example, distal occlusion element 28 is a compliant balloon. As shown in FIG. 11A, first a retrograde guidewire 400 is advanced into a vessel 200 having an occlusion 202. Retrograde guidewire 400 is introduced into vessel 200 from a retrograde direction, via a hollow needle, for example. Next, a guidewire 32 is advanced into vessel 200 from an antegrade direction, as shown in FIG. 11B. Next, catheter 12 having distal occlusion element 28 on inner elongated element 22 is advanced over guidewire 32. In some examples, guidewire 32 is positioned through a lumen of inner elongated element 22. In other examples, guidewire 32 is positioned through a pressure relief element, as described in examples of the present invention. In other examples, guidewire 32 is positioned through outer elongated element lumen 21. As described above, a delivery substance may be introduced through outlet ports 26, 44 during any point in the advancement of catheter 12 into vessel 200. When catheter 12 is in place on the antegrade side of occlusion 202, guidewire 32 is removed from catheter 12, and distal occlusion element 28 is expanded, as shown in FIG. 11D. Next, as shown in FIG. 11E, inner elongated element 22 (or all of catheter 12) is pulled back proximally, shown by arrows 92, and due to the compliant property of distal occlusion element 28, distal occlusion element 28 forms a funnel shape within the vessel 200 and/or within the occlusion 202. Next, as shown in FIG. 11F, retrograde guidewire 400 may be advanced through occlusion 202 and into inner elongated element 22 of catheter 12. Because of the funnel-shape of distal occlusion element 28, it is relatively easy to find the opening in inner elongated element 22. Retrograde guidewire 400 may be comprised of a flexible material and/or design (e.g. coil springs) so as not to puncture distal occlusion element 28. It should be readily apparent that guidewire 32 may alternatively be removed from catheter 12 just before advancement of retrograde guidewire 400. Once retrograde guidewire 400 is in catheter 12, retrograde guidewire 400 may be advanced proximally through catheter 12, and then used to replace guidewire 32 for the procedure to follow.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

## Claims

1. A catheter system comprising:
an outer elongated element (20) having an outer elongated element proximal end (25) and an outer elongated element distal end (27), said outer elongated element (20) having an outer elongated element lumen (21) extending from said outer elongated element proximal end (25) to said outer elongated element distal end (27) and having an outlet port (26) at said outer elongated element distal end (27), and a proximal occlusion element (28) located at said outer elongated element distal end (27), said proximal occlusion element (28) proximal to said outlet port (26), said proximal occlusion element (28) comprising a proximal occlusion element proximal end (70) and a proximal occlusion element distal end (72);
an inner elongated element (22) having an inner elongated element proximal end (16) and an inner elongated element distal end (18), and a distal occlusion element (29) located at said inner elongated element distal end (18), said inner elongated element (22) positioned within said outer elongated element lumen (21) wherein said outer elongated element (20) is coaxially arranged with respect to said inner elongated element (22), said coaxial arrangement providing a delivery lumen (78) between said inner and outer elongated elements (22, 20) for delivery of a delivery substance to a vessel through said delivery lumen (78) and through said outlet port (26), wherein said inner elongated element distal end (18) is distal to and movable with respect to said outer elongated element distal end (27), and wherein said inner elongated element (22) further comprises a pressure relief element (38) at said inner elongated element distal end (18), **characterised in that** said pressure relief element (38) provides controlled removal of blood from the treatment area to a location outside of the treatment area.

2. The catheter system of claim 1, wherein a distance from said proximal occlusion element proximal end (70) to said proximal occlusion element distal end (72) is in a range of 5-40 cm.

3. The catheter system of claim 1, further comprising at least one inflow occlusion element (80) positioned proximal to said proximal occlusion element (28).

4. The catheter system of claim 3, further comprising an inflow occlusion shaft (82) positioned coaxial to said outer elongated element (20), and wherein said at least one inflow occlusion element (80) is positioned on said inflow occlusion shaft.

5. The catheter system of claim 1 wherein said inner elongated element (22) further comprises a core wire extending from and attached to said inner elongated element distal end (18) and extending to and attached to said inner elongated element proximal end (16).

6. The catheter system of claim 5, wherein said core wire is sandwiched between polymeric layers of a shaft of said inner elongated element (22).

7. The catheter system of claim 1, wherein said delivery substance is a therapeutic or diagnostic agent introduced into said outer elongated element lumen (21), and said delivery substance is positioned between an outer wall of said inner elongated element (22) and an inner wall of said outer elongated element (20), and said delivery substance is positioned between said distal and proximal occlusion elements when introduced into a vessel.

8. The catheter system of claim 1, further comprising a hub at said outer elongated element proximal end (25), said hub configured for introducing said delivery substance into said outer elongated element lumen (21)

9. The catheter system of claim 8, wherein said hub is further configured for introducing said inner elongated element (22) through said outer elongated element lumen (21).

10. The catheter system of claim 1, wherein said pressure relief element (38) has an inner diameter which is approximately 0.05mm (0.002") greater than a diameter of a guidewire to be placed therethrough.

11. The catheter system of claim 1, wherein said pressure relief element (38) comprises a separate distal element positioned distal to said distal occlusion element (29).

12. The catheter system of claim 1, wherein said pressure relief element (38) comprises a passageway through said distal occlusion element (29).

13. The catheter system of claim 1, wherein said distal occlusion element (29) is a fixed wire balloon.

14. The catheter system of claim 1, further comprising a guidewire positioned through said outer elongated element (20).

15. The catheter system of claim 1, wherein said inner elongated element (22) comprises an exposed portion having an exposed portion proximal edge at a proximal occlusion element distal end (72) and an exposed portion distal edge at a distal occlusion element proximal end, and wherein said exposed portion has a length extending from said exposed portion proximal edge to said exposed portion distal edge, wherein said length may be varied by moving said inner elongated element (22) with respect to outer elongated element (20).

16. The catheter system of claim 1, further comprising a guidewire positioned within said pressure relief element, wherein a clearance space is provided in said pressure relief element for controlled release of blood from the treatment zone while said guidewire is positioned within said pressure relief element.

## Patentansprüche

1. Kathetersystem, das Folgendes umfasst:
ein äußeres längliches Element (20), das ein proximales Ende (25) des äußeren länglichen Elements und ein distales Ende (27) des äußeren länglichen Elements aufweist, wobei das äußere längliche Element (20) ein Lumen (21) des äußeren länglichen Elements, das sich von dem proximalen Ende (25) des äußeren länglichen Elements bis zu dem distalen Ende (27) des äußeren länglichen Elements erstreckt und eine Auslassöffnung (26) an dem distalen Ende (27) des äußeren länglichen Elements aufweist, ein proximales Verschlusselement (28), das an dem distalen Ende (27) des äußeren länglichen Elements angeordnet ist, aufweist, wobei das proximale Verschlusselement (28) proximal zu der Auslassöffnung (26) liegt, wobei das proximale Verschlusselement (28) ein proximales Ende (70) des proximalen Verschlusselements und ein distales Ende (72) des proximalen Verschlusselements umfasst,
ein inneres längliches Element (22), das ein proximales Ende (16) des inneren länglichen Elements und ein distales Ende (18) des inneren länglichen Elements und ein distales Verschlusselement (29), das an dem distalen Ende (18) des inneren länglichen Elements angeordnet ist, aufweist, wobei das innere längliche Element (22) innerhalb des Lumens (21) des äußeren länglichen Elements angeordnet ist, wobei das äußere längliche Element (20) koaxiale in Bezug auf das innere längliche Element (22) angeordnet ist, wobei die koaxial Anordnung ein Abgabelumen (78) zwischen dem inneren und dem äußeren länglichen Element (22, 20) zur Abgabe einer Abgabesubstanz an ein Gefäß durch das Abgabelumen (78) und durch die Auslassöffnung (26) bereitstellt, wobei das distale Ende (18) des inneren länglichen Elements distal zu dem distalen Ende (27) des äußeren länglichen Elements und beweglich in Bezug auf dasselbe ist und wobei das innere längliche Element (22) ferner ein Druckentlastungselement (38) an dem distalen Ende (18) des inneren länglichen Elements umfasst, **dadurch gekennzeichnet, dass**
das Druckentlastungselement (38) eine kontrollierte Entfernung von Blut aus dem Behandlungsbereich zu einer Stelle außerhalb des Behandlungsbereichs gewährleistet.

2. Kathetersystem nach Anspruch 1, wobei eine Entfernung von dem proximalen Ende (70) des proximalen Verschlusselements zu dem distalen Ende (72) des proximalen Verschlusselements in einem Bereich von 5 bis 40 cm liegt.

3. Kathetersystem nach Anspruch 1, das ferner wenigstens ein Einströmungsverschlusselement (80) umfasst, das proximal zu dem proximalen Verschlusselement (28) angeordnet ist.

4. Kathetersystem nach Anspruch 3, das ferner einen Einströmungsverschlussschaft (82) umfasst, der koaxial zu dem äußeren länglichen Element (20) angeordnet ist, und wobei das wenigstens eine Einströmungsverschlusselement (80) an dem Einströmungsverschlussschaft angeordnet ist.

5. Kathetersystem nach Anspruch 1, wobei das innere längliche Element (22) ferner einen Kerndraht umfasst, der sich von dem distalen Ende (18) des inneren länglichen Elements aus erstreckt und an demselben befestigt ist und sich zu dem proximalen Ende (16) des inneren länglichen Elements erstreckt und an demselben befestigt ist.

6. Kathetersystem nach Anspruch 5, wobei der Kerndraht zwischen Polymerlagen eines Schafts des inneren länglichen Elements (22) eingefügt ist.

7. Kathetersystem nach Anspruch 1, wobei die Abgabesubstanz ein therapeutischer oder diagnostischer Wirkstoff ist, der in das Lumen (21) des äußeren länglichen Elements eingeleitet wird, und die Abgabesubstanz zwischen einer äußeren Wand des inneren länglichen Elements (22) und einer inneren Wand des äußeren länglichen Elements (20) angeordnet ist und die Abgabesubstanz zwischen dem distalen und dem proximalen Verschlusselement angeordnet ist, wenn es in ein Gefäß eingeführt wird.

8. Kathetersystem nach Anspruch 1, das ferner ein Verbindungsstück an dem proximalen Ende (25) des äußeren länglichen Elements umfasst, wobei das Verbindungsstück zum Einleiten der Abgabesubstanz in das Lumen (21) des äußeren länglichen Elements konfiguriert ist.

9. Kathetersystem nach Anspruch 8, wobei das Verbindungsstück ferner zum Einführen des inneren länglichen Elements (22) durch das Lumen (21) konfiguriert ist.

10. Kathetersystem nach Anspruch 1, wobei das Druckentlastungselement (38) einen Innendurchmesser aufweist, der ungefähr 0,05 mm (0,002 ") größer ist als ein Durchmesser eines Führungsdrahts, der durch dasselbe platziert werden soll.

11. Kathetersystem nach Anspruch 1, wobei das Druckentlastungselement (38) ein gesondertes distales Element umfasst, das distal zu dem distalen Verschlusselement (29) angeordnet ist.

12. Kathetersystem nach Anspruch 1, wobei das Druckentlastungselement (38) einen Durchgang durch das distale Verschlusselement (29) umfasst.

13. Kathetersystem nach Anspruch 1, wobei das distale Verschlusselement (29) ein fester Drahtballon ist.

14. Kathetersystem nach Anspruch 1, das ferner einen Führungsdraht umfasst, der durch das äußere längliche Element angeordnet ist (20).

15. Kathetersystem nach Anspruch 1, wobei das innere längliche Element (22) einen freigelegten Abschnitt umfasst, der eine proximale Kante des freigelegten Abschnitts an einem distalen Ende (72) des proximalen Verschlusselements und eine distale Kante des freigelegten Abschnitts an einem proximalen Ende des distalen Verschlusselements aufweist, und wobei der freigelegte Abschnitt eine Länge aufweist, die sich von der proximalen Kante des freigelegten Abschnitts bis zu der distalen Kante des freigelegten Abschnitts erstreckt, wobei die Länge durch Bewegen des inneren länglichen Elements (22) in Bezug auf das äußere längliche Element (20) verändert werden kann.

16. Kathetersystem nach Anspruch 1, das ferner einen Führungsdraht umfasst, der innerhalb des Druckentlastungselements angeordnet ist, wobei ein Freiraum in dem Druckentlastungselement zum kontrollierten Abgeben von Blut aus der Behandlungszone bereitgestellt wird, während der Führungsdraht innerhalb des Druckentlastungselements angeordnet ist.

## Revendications

1. Système de cathéter, comprenant:
un élément externe allongé (209) comportant une extrémité proximale de l'élément externe allongé (25) et une extrémité distale de l'élément externe allongé (27), ledit élément externe allongé (20) comportant une lumière d'élément externe allongé (21) s'étendant de ladite extrémité proximale de l'élément externe allongé (256) vers ladite proximité distale de l'élément externe allongé (27) et comportant une orifice de sortie (26) au niveau de ladite extrémité distale de l'élément externe allongé (27), et un élément d'occlusion proximal (28) agencé au niveau de ladite extrémité distale de l'élément externe allongé (27), ledit élément d'occlusion proximal (28) étant positionné dans une position proximale par rapport audit orifice de sortie (26), ledit élément d'occlusion proximal (28) comprenant une extrémité proximale de l'élément d'occlusion proximal (70) et une extrémité distale de l'élément d'occlusion proximal (72) ;
un élément interne allongé (22) comportant une extrémité proximale de l'élément interne allongé (16) et une extrémité distale de l'élément interne allongé (18), et un élément d'occlusion distal (29) agencé au niveau de ladite extrémité distale de l'élément interne allongé (18), ledit élément interne allongé (22) étant positionné dans ladite lumière de l'élément externe allongé (21), dans lequel ledit élément externe allongé (20) est agencé de manière coaxiale par rapport audit élément interne allongé (22), ledit agencement coaxial établissant une lumière d'administration (78) entre lesdits éléments interne et externe allongés (22, 20) en vue de l'administration d'une substance d'administration à un vaisseau à travers ladite lumière d'administration (78) et à travers ledit orifice de sortie (26), dans lequel ladite extrémité distale de l'élément interne allongé (18) se trouve dans une position distale et peut être déplacée par rapport à ladite extrémité distale de l'élément externe allongé (27), et dans lequel ledit élément interne allongé (22) comprend en outre un élément de détente de pression (38) au niveau de l'extrémité distale de l'élément interne allongé (18), **caractérisé en ce que** :
ledit élément de détente de pression (38) assure une libération contrôlée du sang de la zone de traitement vers un emplacement situé hors de la zone de traitement.

2. Système de cathéter selon la revendication 1, dans lequel une distance entre ladite extrémité proximale de l'élément d'occlusion proximal (70) et ladite extrémité distale de l'élément d'occlusion proximal (72) est comprise dans un intervalle allant de 5 - 40 cm.

3. Système de cathéter selon la revendication 1, comprenant en outre au moins un élément d'occlusion d'entrée (80) positionné dans une position proximale par rapport audit élément d'occlusion proximal (28).

4. Système de cathéter selon la revendication 3, comprenant en outre un arbre d'occlusion d'entrée (82) positionné de manière coaxiale par rapport audit élément externe allongé (20), et dans lequel ledit au moins un élément d'occlusion d'entrée (80) est positionné sur ledit arbre d'occlusion d'entrée.

5. Système de cathéter selon la revendication 1, dans lequel ledit élément interne allongé (22) comprend en outre un fil central s'étendant à partir de l'extrémité distale dudit élément interne allongé (18) et fixé sur celui-ci, et s'étendant vers ladite extrémité proximale de l'élément interne allongé (16) et fixé sur celui-ci.

6. Système de cathéter selon la revendication 5, dans lequel ledit fil central est pris en sandwich entre des couches polymères d'un arbre dudit élément interne allongé (22).

7. Système de cathéter selon la revendication 1, dans lequel ladite substance d'administration est un agent thérapeutique ou diagnostique introduit dans ladite lumière de l'élément externe allongé (21), ladite substance d'administration étant positionnée entre une paroi externe dudit élément interne allongé (22) et une paroi interne dudit élément externe allongé (20), ladite substance d'administration étant positionnée entre lesdits éléments d'occlusion distal et proximal lors de l'introduction dans un vaisseau.

8. Système de cathéter selon la revendication 1, comprenant en outre un moyeu au niveau de ladite extrémité proximale de l'élément externe allongé (25), ledit moyeu étant configuré pour introduire ladite substance d'administration dans ladite lumière de l'élément externe allongé (21).

9. Système de cathéter selon la revendication 8, dans lequel ledit moyeu est en outre configuré pour introduire ledit élément interne allongé (22) à travers ladite lumière de l'élément externe allongé (21).

10. Système de cathéter selon la revendication 1, dans lequel ledit élément de détente de pression (38) a un diamètre intérieur correspond à peu près à 0,05 mm (0,002"), supérieur à un diamètre d'un fil-guide devant être placé à travers celui-ci.

11. Système de cathéter selon la revendication 1, dans lequel ledit élément de détente de pression (38)) comprend un élément distal séparé positionné en une position distale par rapport audit élément d'occlusion distal (29).

12. Système de cathéter selon la revendication 1, dans lequel ledit élément de détente de pression (38) comprend un passage traversant ledit élément d'occlusion distal (29).

13. Système de cathéter selon la revendication 1, dans lequel ledit élément d'occlusion distal (29) est un ballonnet en fil métallique fixe.

14. Système de cathéter selon la revendication 1, comprenant en outre un fil-guide positionné à travers ledit élément externe allongé (20).

15. Système de cathéter selon la revendication 1, dans lequel ledit élément interne allongé (22) comprend une partie exposée comportant un bord proximal de la partie exposée au niveau d'une extrémité distale de l'élément d'occlusion proximal et un bord distal de la partie exposée au niveau de l'extrémité proximale de l'élément d'occlusion distal, et dans lequel ladite partie exposée a une longueur s'étendant de dudit bord proximal de ladite partie exposée vers ledit bord distal de ladite partie exposée, ladite longueur pouvant être changée en déplaçant ledit élément interne allongé (22) par rapport audit élément externe allongé (20).

16. Système de cathéter selon la revendication 1, comprenant en outre un fil-guide positionné dans ledit élément de détente de pression, dans lequel un espace de dégagement est établi dans ledit élément de détente de pression en vue d'une libération contrôlée du sang de la zone de traitement, pendant que ledit fil-guide est positionné dans ledit élément de détente de pression.
